# EUROPÄISCHE PATENTANMELDUNG

(11) **EP 3 738 643 A1**
(43) Veröffentlichungstag der Anmeldung: **18.11.2020**
(21) Anmeldenummer: 19174375.6
(22) Anmeldetag: 14.05.2019
(51) Int. Cl.: A61N 1/05, A61N 1/375, A61N 1/362

(54) **CRT-SYSTEM MIT EINEM PULSGENERATOR UND EINER ADAPTEREINRICHTUNG**

(71) Anmelder: BIOTRONIK SE & Co. KG, 12359 Berlin (DE)
(72) Erfinder: Dörr, Thomas, 12437 Berlin (DE); Lang, Volker, 12161 Berlin (DE)
(74) Vertreter: Keck, Hans-Georg

(57) **Zusammenfassung**

Ein CRT-System (1) zur kardialen Resynchronisationstherapie umfasst einen Pulsgenerator (10), der einen Anschlussblock (101) mit einer Mehrzahl von Steckverbinderanschlüssen (102-104) aufweist, und eine Mehrzahl von an die Steckverbinderanschlüsse (102-104) anschließbaren Elektroden (11, 12, 12A, 12B, 13), die jeweils einen Gegensteckverbinder (112, 122, 122A, 122B, 132) aufweisen, Zusätzlich ist eine Adaptereinrichtung (2) vorgesehen, die einen ersten Steckverbinder (21) und zumindest zwei zweite Steckverbinder (22, 23) aufweist, wobei der erste Steckverbinder (21) steckend mit einem der Steckverbinderanschlüsse (102-104) des Anschlussblocks (101) des Pulsgenerators (10) und die zweiten Steckverbinder (22, 23) jeweils steckend mit einem Gegensteckverbinder (112, 122, 122A, 122B, 132) einer der Mehrzahl von Elektroden (11, 12, 12A, 12B, 13) verbindbar sind.

## Beschreibung

Die vorliegende Erfindung betrifft ein CRT-System zur kardialen Resynchronisationstherapie.

Ein derartiges CRT-System umfasst einen Pulsgenerator, der einen Anschlussblock mit einer Mehrzahl von Steckverbinderanschlüssen aufweist, und eine Mehrzahl von an die Steckverbinderanschlüsse anschließbaren Elektroden, die jeweils einen Gegensteckverbinder aufweisen.

Ein solches CRT-System dient zur sogenannten kardialen Resynchronisationstherapie (Englisch "Cardiac Resynchronisation Therapy", kurz CRT). Bei einigen Patienten mit einer chronischen Herzschwäche (so genannter Herzinsuffizienz) ist die natürliche Erregung zur Kontraktion der Herzkammern gestört. Bei einem sogenannten Linksschenkelblock kann hierbei insbesondere die Erregung der linken Herzkammer (linker Ventrikel) derart beeinträchtigt sein, dass die linke Herzkammer nur unzureichend und zudem nicht synchron mit der rechten Herzkammer kontrahiert. Eine Folge ist, dass die Herzkammern nicht mehr in einer aufeinander abgestimmten Weise arbeiten, das Herz somit unregelmäßig schlägt und dadurch die Pumpleistung des Herzens reduziert ist.

Ziel der kardialen Resynchronisationstherapie ist es somit, die Erregung der Herzkammern so zu beeinflussen, dass die Synchronität zwischen den Herzkammern wiederhergestellt wird, indem die rechte und linke Herzkammer durch eingespeiste elektrische Erregung zeitgleich stimuliert werden.

Ein CRT-System verwendet zu diesem Zweck mehrere Elektroden, die gemeinsam mit dem Pulsgenerator implantiert und von denen eine in die rechte Herzkammer (rechter Ventrikel) und eine andere in die linke Herzkammer (linker Ventrikel) verlegt werden. Eine dritte Elektrode kann zusätzlich im rechten Vorhof (rechtes Atrium) angeordnet sein, um eine Abstimmung mit einer Vorhofaktivität zu ermöglichen.

Ein solches CRT-System kann mit einer reinen Herzschrittmacherfunktion ausgestaltet sein (sogenanntes CRT-P-System). Weil Patienten mit einer chronischen Herzschwäche häufig auch einem erhöhten Risiko eines plötzlichen Herzstillstands (plötzlicher Herztod) unterliegen, kann ein CRT-System zusätzlich aber auch eine Defibrillator-Funktion zur hochenergetischen Anregung zum Zwecke der Defibrillation aufweisen (sogenanntes CRT-D-System).

Wünschenswert bei solchen CRT-Systemen ist die Möglichkeit für eine flexible, auf spezifische Symptome eines Patienten abgestimmte Stimulation. Während bestehende CRT-Systeme definierte Anschlüsse zum Anschließen bestimmter Elektroden, die in bestimmungsgemäßer Weise beispielsweise in die linke Herzkammer, die rechte Herzkammer oder das rechte Atrium zu implantieren sind, aufweisen, kann wünschenswert sein, auch andere Elektroden an bestehenden CRT-Systemen zu verwenden. Dies ist mit herkömmlichen CRT-Systemen nicht ohne weiteres möglich.

Aufgabe der vorliegenden Erfindung ist es, ein CRT-System zur Verfügung zu stellen, dass in flexibler Weise das Anschließen unterschiedlicher Elektroden ermöglicht.

Diese Aufgabe wird durch einen Gegenstand mit den Merkmalen des Anspruchs 1 gelöst.

Demnach weist das CRT-System eine Adaptereinrichtung auf, die einen ersten Steckverbinder und zumindest zwei zweite Steckverbinder umfasst. Der erste Steckverbinder kann steckend mit einem der Steckverbinderanschlüsse des Anschlussblocks des Pulsgenerators verbunden werden. Die zweiten Steckverbinder können demgegenüber jeweils steckend mit einem Gegensteckverbinder einer der Mehrzahl von Elektroden verbunden werden.

Herkömmlich sind die Elektroden über ihre Gegensteckverbinder unmittelbar an die Steckverbinderanschlüsse des Anschlussblocks des Pulsgenerators anzuschließen. Die Steckverbinderanschlüsse des Pulsgenerators sind hierbei herkömmlich vorbestimmten Elektroden, beispielsweise einer Elektrode zur linksventrikulären Stimulation, einer Elektrode zur rechtsventrikulären Stimulation und einer Elektrode zur Stimulation im rechten Atrium, zugeordnet. Über eine Steuereinrichtung des Pulsgenerators werden die Steckverbinderanschlüsse und darüber die angeschlossenen Elektroden angesteuert, um elektrische Stimulationspulse in bestimmungsgemäßer Weise abzugeben.

Vorliegend wird vorgeschlagen, zusätzlich eine Adaptereinrichtung zu verwenden, die zwischen den Pulsgenerator und zumindest einige der Elektroden geschaltet werden kann. Die Adaptereinrichtung kann mit einem der Steckverbinderanschlüsse des Anschlussblocks des Pulsgenerators steckend verbunden werden, um auf diese Weise mit dem Pulsgenerator elektrisch zu kontaktieren. Über zwei (oder mehr) zweite Steckverbinder können zwei (oder mehr) Elektroden an die Adaptereinrichtung und darüber an den zugeordneten Steckverbinderanschluss des Pulsgenerators angeschlossen werden, sodass zwei (oder mehr) Elektroden mit einem einzigen Steckverbinderanschluss des Anschlussblocks des Pulsgenerators verschaltet werden können.

Insbesondere kann auf diese Weise ermöglicht werden, an einen Steckverbinderanschluss, der bei einem herkömmlichen CRT-System zum Anschluss einer multipolaren linksventrikulären Elektrode vorgesehen und bestimmt ist, zwei Elektroden anzuschließen, von denen eine beispielsweise zur linksventrikulären Stimulation und eine andere zur sogenannten HIS-Bündel-Stimulation ausgelegt und entsprechend im Herzen eines Patienten zu implantieren ist. Über einen herkömmlich zur linksventrikulären Stimulation vorgesehenen Steckverbinderanschluss des Anschlussblocks des Pulsgenerators kann somit (auch) eine HIS-Bündel-Stimulation bewirkt werden.

Die Adaptereinrichtung ermöglicht die Verwendung eines herkömmlichen CRT-Pulsgenerators (auch) zur HIS-Bündel-Stimulation. Im Rahmen einer solchen HIS-Bündel-Stimulation wird Stimulationsenergie im Bereich des sogenannten HIS-Bündels im Septum zwischen den Herzkammern (Ventrikeln) eingespeist. Das HIS-Bündel schließt an den sogenannten atrioventrikulären (AV) Knoten an und teilt sich in einen linken Bündelzweig, der sich in den linken Ventrikel erstreckt, und einen rechten Bündelzweig, der sich in den rechten Ventrikel erstreckt. Durch eine Stimulation am HIS-Bündel ist eine synchrone Erregung für eine synchrone Arbeitsweise der Ventrikel möglich.

Weil elektrische Stimulationspulse für eine linksventrikuläre Stimulation und für eine HIS-Bündel-Stimulation in ihrer Art miteinander vergleichbar sind, ist eine solche Verwendung eines herkömmlichen CRT-Systems zusammen mit einer HIS-Bündel-Elektrode möglich, ohne dass besondere Anpassungen am Pulsgenerator des CRT-Systems vorgenommen werden müssen. Über die Adaptereinrichtung kann die Verwendungsmöglichkeit des CRT-Systems somit flexibel erweitert werden, für eine ergänzende Stimulation an anderen Stimulationsorten, über die für ein herkömmliches CRT-System definierten Stimulationsorte hinaus.

In einer Ausgestaltung weist der erste Steckverbinder erste elektrische Kontaktelemente zum elektrischen Kontaktieren mit Kontaktelementen des zugeordneten Steckverbinderanschlusses des Anschlussblocks des Pulsgenerators auf. Über die elektrischen Kontaktelemente kann der erste Steckverbinder der Adaptereinrichtung elektrisch mit zugeordneten elektrischen Kontaktelementen des zugeordneten Steckverbinderanschlusses des Pulsgenerators kontaktieren, sodass über den Steckverbinderanschluss elektrische Signale in die Adaptereinrichtung eingeleitet werden können.

Der erste Steckverbinder kann beispielsweise vierpolig mit vier elektrischen Kontaktelementen ausgestaltet sein. Entsprechend kann der erste Steckverbinder an einen vierpoligen Steckverbinderanschluss des Anschlussblocks des Pulsgenerators angeschlossen werden. Beispielsweise ist der erste Steckverbinder an einen vierpoligen Steckverbinderanschluss des Anschlussblocks anzuschließen, der herkömmlich für eine linksventrikuläre Stimulation über eine linksventrikulär verlegte Elektrode vorgesehen ist.

Der erste Steckverbinder kann genormt sein und entsprechend an einen genormten Steckverbinderanschluss des Anschlussblocks anzuschließen sein. Beispielsweise kann es sich bei dem ersten Steckverbinder um einen sogenannten IS4-Stecker, der nach der Norm ISO 27186:2010 (erste Ausgabe aus dem März 2010) genormt ist, oder einen DF4-Stecker handeln. Ist der erste Steckverbinder als DF4-Stecker ausgebildet, kann eine HIS-Bündel-Elektrode einem RV-Stimulationskanal zugeordnet werden, und es könnten separate Schockelektroden implantiert werden.

In einer Ausgestaltung weisen die zweiten Steckverbinder der Adaptereinrichtung jeweils zweite elektrische Kontaktelemente zum elektrischen Kontaktieren mit Kontaktelementen des Gegensteckverbinders der zugeordneten Elektrode auf. Ein jeder zweiter Steckverbinder der Adaptereinrichtung dient zum steckenden Verbinden mit einem zugeordneten Gegensteckverbinder einer der Elektroden, wobei über die zweiten elektrischen Kontaktelemente des zweiten Steckverbinders eine elektrische Kontaktierung zwischen der Elektrode und der Adaptereinrichtung hergestellt wird.

Ein oder mehrere der zweiten Steckverbinder können als zweipoliger Steckverbinder mit zwei zweiten elektrischen Kontaktelementen ausgestaltet sein. In diesem Fall kann es sich bei dem zweiten Steckverbinder beispielsweise um einen zweipoligen IS-1-Anschluss (nach ISO 27186:2010) handeln.

Andere der zweiten Steckverbinder können beispielsweise vierpolig mit vier zweiten elektrischen Kontaktelementen ausgestaltet und als IS4-Anschluss (nach ISO 27186:2010) verwirklicht sein.

Denkbar und möglich ist auch, dass einer oder mehrere der zweiten Steckverbinder als einpolige Steckverbinder mit jeweils nur einem elektrischen Kontaktelement ausgebildet sind, beispielsweise in Form einpoliger IS-1-Anschlüsse (nach ISO 27186:2010).

In einer konkreten Ausgestaltung kann die Adaptereinrichtung genau zwei zweite Steckverbinder aufweisen. In diesem Fall ist die Adaptereinrichtung als sogenanntes Y-Stück verwirklicht, bei dem der eine erste Steckverbinder mit zwei zweiten Steckverbindern verbunden und elektrisch auf die zwei zweiten Steckverbinder verteilt wird. In diesem Fall können beispielsweise beide zweite Steckverbinder als (einpolige oder zweipolige) IS-1-Anschlüsse ausgebildet sein. Alternativ können beide zweite Steckverbinder als vierpolige IS4-Anschlüsse ausgebildet sein. Wiederum alternativ kann einer der zweiten Steckverbinder als vierpoliger IS4-Anschluss und der andere der zweiten Steckverbinder als einpoliger oder zweipoliger IS-1-Anschluss ausgestaltet sein.

Entsprechend ihrer Ausgestaltung mit einem, zwei oder vier Kontaktelementen können die IS-1- bzw. IS4-Anschlüsse der zweiten Steckverbinder mit IS-1- oder IS4-Steckern zugeordneter Elektroden verbunden werden.

In einer Ausgestaltung sind zumindest einige der zweiten elektrischen Kontaktelemente der zweiten Steckverbinder der Adaptereinrichtung mit zugeordneten elektrischen Kontaktelementen des ersten Steckverbinders elektrisch verbunden. In einer konkreten Ausgestaltung sind sämtliche der zweiten elektrischen Kontaktelemente der zweiten Steckverbinder mit zugeordneten ersten elektrischen Kontaktelementen des ersten Steckverbinders elektrisch verbunden. Die zweiten elektrischen Kontaktelemente eines jeden der zweiten Steckverbinder können hierbei mit unterschiedlichen ersten Kontaktelementen des ersten Steckverbinders verbunden sein, sodass eine definierte Zuordnung von ersten elektrischen Kontaktelementen des ersten Steckverbinders zu zweiten elektrischen Kontaktelementen der zweiten Steckverbinder vorliegt.

Beispielsweise können zwei erste elektrische Kontaktelemente des zum Beispiel vierpolig ausgebildeten ersten Steckverbinders mit zwei zweiten elektrischen Kontaktelementen eines der zweiten Steckverbinder verbunden sein. Die anderen beiden der ersten elektrischen Kontaktelemente des vierpolig ausgebildeten ersten Steckverbinders sind in diesem Fall beispielsweise mit zwei zweiten elektrischen Kontaktelementen des anderen zweiten Steckverbinders verschaltet.

Über den zum Beispiel vierpolig ausgebildeten ersten Steckverbinder, der mit einem zugeordneten Steckverbinderanschluss des Anschlussblocks des Pulsgenerators zum Zwecke der elektrischen Kontaktierung steckend zu verbinden ist, können somit selektiv die zweiten Steckverbinder und somit die daran angeschlossenen Elektroden angesteuert werden, um über die angeschlossenen Elektroden elektrische Stimulationspulse abzugeben.

In einer Ausgestaltung ist eine der Elektroden, die an einen der zweiten Steckverbinder der Adaptereinrichtung anzuschließen ist, als HIS-Bündel-Elektrode zur Stimulation am HIS-Bündel eines Patienten ausgebildet und entsprechend im Herzen zu implantieren. Eine andere Elektrode, die an einen anderen der zweiten Steckverbinder anzuschließen ist, kann demgegenüber beispielsweise zur linksventrikulären Stimulation ausgebildet sein. Ist die Adaptereinrichtung an einen Steckverbinderanschluss des Anschlussblocks des Pulsgenerators anzuschließen, der bei herkömmlicher Verwendung für eine linksventrikuläre Stimulation vorgesehen und bestimmt ist, kann über diesen einen Steckverbinderanschluss somit, durch Verwendung der Adaptereinrichtung, eine Stimulation an unterschiedlichen Stimulationsorten, nämlich am HIS-Bündel sowie linksventrikulär ermöglicht werden.

Bei einem herkömmlichen vierpoligen Steckverbinderanschluss des Anschlussblocks des Pulsgenerators, der für eine linksventrikuläre Stimulation vorgesehen und bestimmt ist, kann eine zeitliche Verzögerung zwischen während eines Herzzyklus in den linken Ventrikel einzuleitenden Stimulationspulsen über ein sogenanntes LV1-LV2-Delay eingestellt werden. Eine solche zeitliche Verzögerung kann an dem Pulsgenerator programmierbar und adaptiv veränderbar sein. Über einen solchen Verzögerungsparameter kann, bei Verwendung der Adaptereinrichtung, eine zeitliche Verzögerung zwischen einer Stimulation am HIS-Bündel (über eine an einen der zweiten Steckverbinder angeschlossene HIS-Bündel-Elektrode) und einer linksventrikulären Stimulation (über eine an einen anderen zweiten Steckverbinder angeschlossene linksventrikuläre Elektrode) eingestellt werden.

Die Adaptereinrichtung ist vorteilhafterweise gemeinsam mit dem Pulsgenerator und den Elektroden in einem Patienten zu implantieren. Der Pulsgenerator wird hierbei beispielsweise subkutan im Bereich des Schlüsselbeins in einem Patienten implantiert. Die Adaptereinrichtung ist, im implantierten Zustand, mit dem Pulsgenerator verbunden und erstreckt sich von dem Pulsgenerator. Elektroden wiederum sind an den Pulsgenerator bzw. die Adaptereinrichtung angeschlossen und erstrecken sich zum Herzen und sind entsprechend ihrer Bestimmung hin zu zugeordneten Stimulationsorten verlegt.

Die Aufgabe wird auch gelöst durch ein Verfahren zur Durchführung einer kardialen Resynchronisationstherapie unter Verwendung eines CRT-Systems, das aufweist: Bereitstellen eines Pulsgenerators des CRT-Systems, der einen Anschlussblock mit einer Mehrzahl von Steckverbinderanschlüssen aufweist; Bereitstellen einer Mehrzahl von an die Steckverbinderanschlüsse anschließbaren Elektroden, die jeweils einen Gegensteckverbinder aufweisen; Verbinden eines ersten Steckverbinders einer Adaptereinrichtung mit einem der Steckverbinderanschlüsse des Anschlussblocks des Pulsgenerators; und Verbinden der Gegensteckverbinder von zumindest zwei der Mehrzahl von Elektroden mit zweiten Steckverbindern der Adaptereinrichtung.

Die vorangehend für das CRT-System beschriebenen Vorteile und vorteilhaften Ausgestaltungen finden analog auch auf das Verfahren Anwendung.

Das Verfahren zur kardialen Resynchronisationstherapie ermöglicht die Verwendung eines herkömmlichen, für eine kardiale Resynchronisationstherapie eingesetzten Pulsgenerators unter zusätzlicher Verwendung einer HIS-Bündel-Elektrode zur Stimulation am HIS-Bündel des Herzens eines Patienten. Über die Adaptereinrichtung kann eine solche HIS-Bündel-Elektrode beispielsweise an einen Steckverbinderanschluss des Pulsgenerators angeschlossen werden, der herkömmlich für eine linksventrikuläre Stimulation verwendet wird. Die Adaptereinrichtung ermöglicht hierbei über ihre zweiten Steckverbinder ein Anschließen von mehreren Elektroden, beispielsweise einer HIS-Bündel-Elektrode und einer linksventrikulär zu platzierenden Elektrode, an einen Steckverbinderanschluss des Pulsgenerators.

In einer Ausgestaltung wird eine erste Elektrode der Mehrzahl von Elektroden als HIS-Bündel-Elektrode mit einem distalen Ende im Bereich des HIS-Bündels des Herzens eines Patienten implantiert. Eine solche HIS-Bündel-Elektrode kann beispielsweise über die Adaptereinrichtung an den Pulsgenerator angeschlossen werden, indem die HIS-Bündel-Elektrode mit ihrem Gegensteckverbinder an einen zweiten Steckverbinder der Adaptereinrichtung angeschlossen und darüber mit dem Pulsgenerator verbunden wird.

In weiterer Ausgestaltung können eine zweite Elektrode mit ihrem distalen Ende im rechten Atrium, eine dritte Elektrode mit ihrem distalen Ende im linken Ventrikel und/oder eine vierte Elektrode mit ihrem distalen Ende im rechten Ventrikel implantiert werden. Unter Verwendung der Adaptereinrichtung können beispielsweise vier Elektroden an den Pulsgenerator angeschlossen werden, wobei über die Elektroden eine Stimulation im linken Ventrikel, im rechten Ventrikel und zusätzlich am HIS-Bündel erfolgen und zusätzlich beispielsweise eine Vorhofaktivität im rechten Atrium detektiert werden kann.

Im Betrieb gibt der Pulsgenerator Stimulationspulse ab und wertet zudem empfangene Signale, beispielsweise über eine im rechten Atrium implantierte Elektrode, aus, um eine Stimulation für eine synchrone Anregung an unterschiedlichen Orten im Herzen zu bewirken.

Der der Erfindung zugrunde liegende Gedanke soll nachfolgend anhand der in den Figuren dargestellten Ausführungsbeispiele näher erläutert werden. Es zeigen:
- Fig. 1: eine Ansicht des menschlichen Herzens mit einem implantierten CRT-System;
- Fig. 2: eine schematische Ansicht eines Anschlussblocks eines Pulsgenerators eines CRT-Systems;
- Fig. 3: eine Ansicht eines Ausführungsbeispiels einer Adaptereinrichtung zum Verbinden mit einem Pulsgenerator;
- Fig. 4: eine Ansicht der Adaptereinrichtung, darstellend eine elektrische Verschaltung zwischen Kontaktelementen eines ersten Steckverbinders und Kontaktelementen zweier zweiter Steckverbinder;
- Fig. 5: eine schematische Ansicht eines anderen Ausführungsbeispiels einer Adaptereinrichtung;
- Fig. 6: eine schematische Ansicht eines wiederum anderen Ausführungsbeispiels einer Adaptereinrichtung; und
- Fig. 7: eine Ansicht des menschlichen Herzens mit implantierten Elektroden.

Im folgenden werden Ausführungsbeispiele der vorliegenden Erfindung mit Bezug auf die Figuren beschrieben. In den Figuren sind hierbei Bauteile gleicher Funktion mit gleichen Bezugszeichen versehen.

Bereits an dieser Stelle sei angemerkt, dass die beschriebenen Ausführungsbeispiele nicht als beschränkend für die vorliegende Erfindung zu verstehen sind, sondern lediglich der Illustration dienen.

Im Rahmen der vorliegenden Erfindung wird vorgeschlagen, ein CRT-System zur kardialen Resynchronisationstherapie durch Einsatz einer Adaptereinrichtung flexibel mit unterschiedlichen Elektroden zu verwenden, um eine Stimulation an unterschiedlichen Stimulationsorten zu ermöglichen.

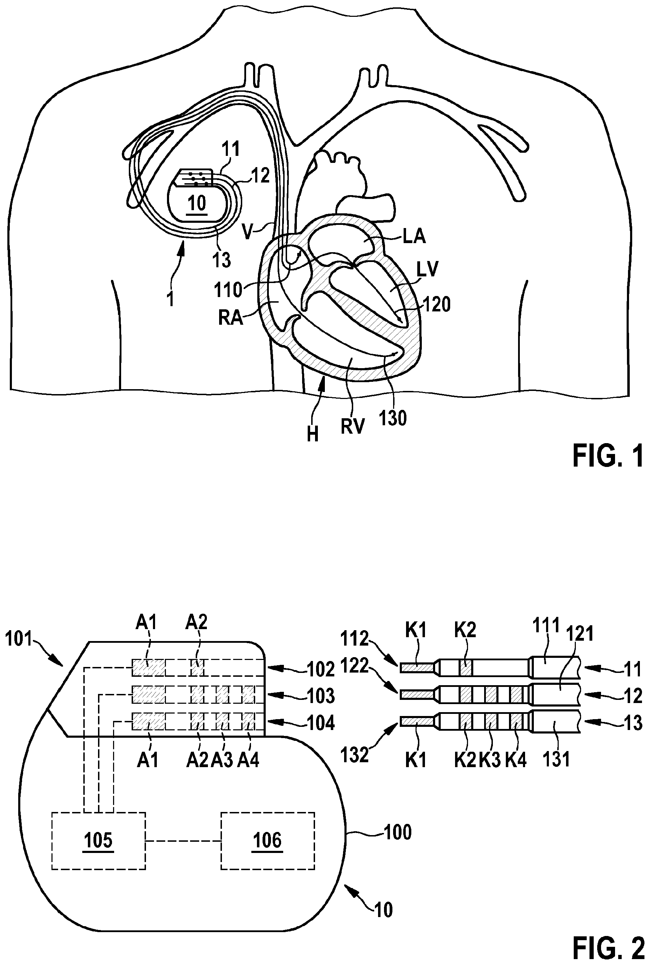

Fig. 1 zeigt in einer schematischen Ansicht das Herz H eines Patienten mit einem implantierten CRT-System 1. Das CRT-System 1 weist einen Pulsgenerator 10 auf, an den Elektroden 11, 12, 13 angeschlossen sind. Der Pulsgenerator 10 ist zusammen mit den Elektroden 11, 12, 13 im Patienten implantiert derart, dass sich die Elektroden 11, 12, 13 ausgehend vom Pulsgenerator 10 durch eine obere Hohlvene V hin zum Herzen H erstrecken und zu unterschiedlichen Stimulationsorten im Herzen H verlegt sind.

Bei dem dargestellten Beispiel sind drei Elektroden 11, 12, 13 an den Pulsgenerator 10 angeschlossen. Der Pulsgenerator 10 ist beispielsweise subkutan im Bereich des Schlüsselbeins des Patienten implantiert. Von dem Pulsgenerator 10 sind die Elektroden 11, 12, 13 derart verlegt, dass die Elektroden 11, 12, 13 mit ihren distalen Enden 110, 120, 130 im rechten Atrium RA (Elektrode 11 mit dem distalen Ende 110), im linken Ventrikel LV (Elektrode 12 mit dem distalen Ende 120) und im rechten Ventrikel RV (Elektrode 13 mit dem distalen Ende 130) zu liegen kommen und somit über durch den Pulsgenerator 10 generierte und in die Elektroden 11, 12, 13 eingeleitete Stimulationspulse eine Stimulation an unterschiedlichen Stimulationsorten im Herzen H erfolgen kann.

Im Rahmen der kardialen Resynchronisationstherapie erfolgt eine Stimulation im linken Ventrikel LV und im rechten Ventrikel RV, sodass durch die Stimulation eine Synchronität der Ventrikelaktivität bewirkt werden kann. Auf diese Weise soll bei Patienten mit einer chronischen Herzschwäche (Herzinsuffizienz) die Pumpleistung des Herzens H erhöht werden.

Wie schematisch in einem Ausführungsbeispiel in Fig. 2 dargestellt ist, weist der Pulsgenerator 10 ein Gehäuse 100 auf, in dem elektrische und elektronische Komponenten in Form einer Steuereinrichtung 105 und einer Energieversorgungseinrichtung 106 in Form einer Batterie eingefasst und gekapselt sind.

An dem Gehäuse 100 ist ein Anschlussblock 101 angeordnet, der Steckverbinderanschlüsse 102, 103, 104 in Form von Buchsenverbindern aufweist, in die die Elektroden 11, 12, 13 mit zugeordneten Gegensteckverbindern 112, 122, 132 in Form von Steckern eingesteckt werden können, um eine elektrische Kontaktierung zwischen den Elektroden 11, 12, 13 und den Steckverbinderanschlüssen 102, 103, 104 und somit mit dem Pulsgenerator 10 zu erreichen.

Die Steckverbinderanschlüsse 102, 103, 104 sind vorzugsweise genormt und sind beispielsweise als IS-1- oder IS4-Anschlüsse nach ISO 27186:2010 ausgestaltet. Die Steckverbinderanschlüsse 102, 103, 104 weisen jeweils elektrische Kontaktelemente A1-A4 auf, wobei ein erster Steckverbinderanschluss 102 beispielsweise zwei elektrische Kontaktelemente A1, A2 und die anderen beiden Steckverbinderanschlüsse 103, 104 jeweils vier elektrische Kontaktelemente A1-A4 aufweisen können. Der Steckverbinderanschluss 102 ist somit als zweipoliger Anschluss ausgestaltet, während die anderen Steckverbinderanschlüsse 103, 104 als vierpolige Anschlüsse ausgebildet sind.

Entsprechend der Ausgestaltung der Steckverbinderanschlüsse 102, 103, 104 sind die Gegensteckverbinder 112, 122, 132 der Elektroden 11, 12, 13 beispielsweise als IS-1-Stecker (Gegensteckverbinder 112) oder IS4-Stecker (Gegensteckverbinder 122, 132) ausgestaltet und weisen zwei elektrische Kontaktelemente K1, K2 (zweipoliger Gegensteckverbinder 112) oder vier elektrische Kontaktelemente K1-K4 (vierpolige Gegensteckverbinder 122, 132) auf.

Die Kontaktelemente A1-A4 auf Seiten der Steckverbinderanschlüsse 102, 103, 104 und die Kontaktelemente K1-K4 auf Seiten der Gegensteckverbinder 112, 122, 132 sind derart ausgestaltet, dass beim steckenden Verbinden eine paarweise elektrische Kontaktierung zwischen den jeweils zugeordneten Kontaktelementen A1-A4, K1-K4 hergestellt wird.

Im Rahmen eines herkömmlichen CRT-Systems sind die Steckverbinderanschlüsse 102, 103, 104 jeweils bestimmten Elektroden 11, 12, 13 zugeordnet. So dient ein Steckverbinderanschluss 102 zum Anschließen einer Elektrode 11, die in das rechte Atrium RA zu verlegen ist. Ein zweiter Steckverbinderanschluss 103 dient demgegenüber zum Anschließen einer linksventrikulären Elektrode 12. Ein Steckverbinderanschluss 104 dient zum Anschließen einer rechtsventrikulären Elektrode 13.

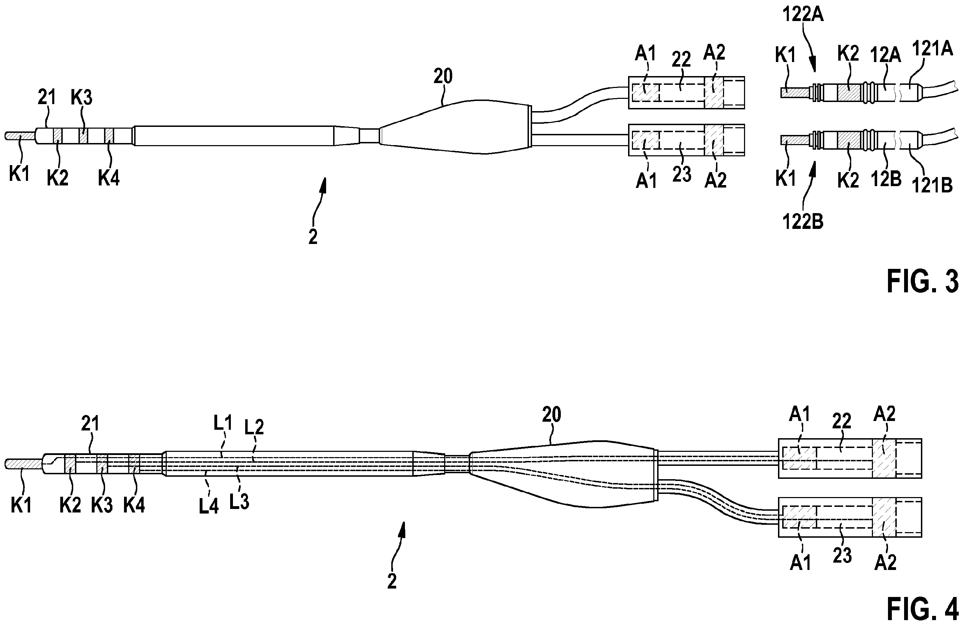

Im Rahmen der vorliegenden Erfindung wird vorgeschlagen, zusammen mit einem Pulsgenerator 10 eines CRT-Systems 1 eine Adaptereinrichtung 2 zu verwenden, wie sie in einem Ausführungsbeispiel in Fig. 3 dargestellt ist. Die Adaptereinrichtung 2 kann mit einem ersten Steckverbinder 21 in Form eines Steckers an einen der Steckverbinderanschlüsse 102, 103, 104 des Anschlussblocks 101 des Pulsgenerators 10 angeschlossen werden und weist zwei zweite Steckverbinder 22, 23 auf, die über ein Verbindungsstück 20 mit dem ersten Steckverbinder 21 verbunden sind und ein Anschließen zweier Elektroden 12A, 12B ermöglichen. Über die Adaptereinrichtung 2 kann somit eine Verteilung derart vorgenommen werden, dass an einen einzigen Steckverbinderanschluss 102, 103, 104 zwei gesonderte Elektroden 12A, 12B angeschlossen werden können.

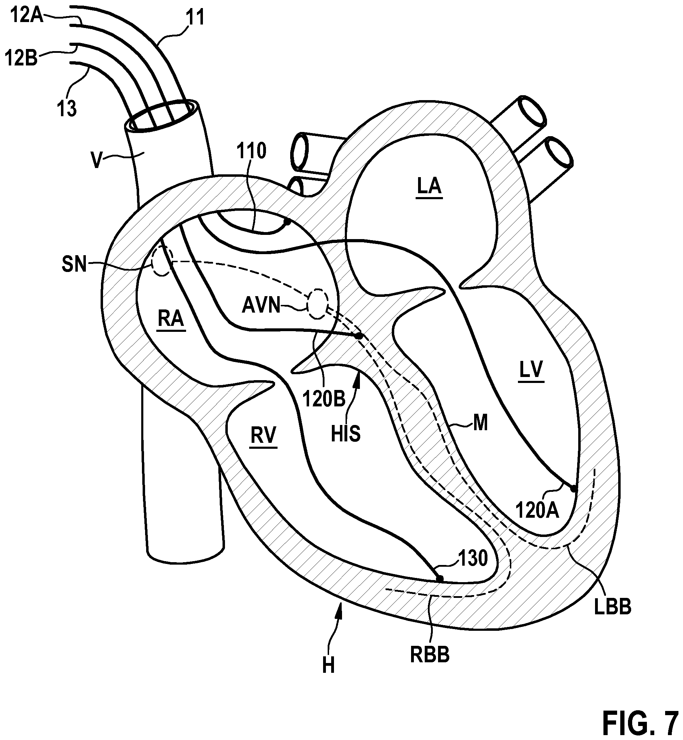

Beispielsweise kann die Adaptereinrichtung 2 an den Steckverbinderanschluss 103 angeschlossen werden, der herkömmlich zum Anschließen einer linksventrikulären Elektrode 12 (siehe Fig. 2) vorgesehen und bestimmt ist. Über die Adaptereinrichtung 2 können somit an den linksventrikulären Steckverbinderanschluss 103 zwei Elektroden 12A, 12B angeschlossen werden, von denen - wie dies in Fig. 7 dargestellt ist - eine Elektrode 12A mit einem distalen Ende 120A über das linke Atrium LA im linken Ventrikel LV zu implantieren ist, während die andere Elektrode 12B derart zum Beispiel in das rechte Atrium RA erstreckt ist, dass das distale Ende 120B der Elektrode 12B im Bereich des HIS-Bündels HIS für eine HIS-Bündel-Stimulation zu liegen kommt.

Die Adaptereinrichtung 2 kann somit bei einem herkömmlichen CRT-System eine zusätzliche Stimulation im Bereich des HIS-Bündels HIS ermöglichen. Das HIS-Bündel HIS ist - wie in Fig. 7 dargestellt - im Septum zwischen den Ventrikeln LV, RV (Myokard M) angeordnet, schließt an den dem Sinusknoten SN nachgeordneten AV-Knoten an und teilt sich in den linken Bündelzweig LBB und den rechten Bündelzweig RBB, die sich um den linken Ventrikel LV bzw. den rechten Ventrikel RV herumstrecken. Durch Anregung am HIS-Bündel HIS können der linke Bündelzweig LBB und der rechte Bündelzweig RBB synchron angeregt werden, sodass Reize synchron in den linken Ventrikel LV und den rechten Ventrikel RV eingeleitet werden können.

Im Rahmen einer kardialen Resynchronisationstherapie kann somit eine Stimulation insbesondere für eine synchrone Anregung des linken Ventrikels LV und des rechten Ventrikels RV flexibler gestaltet und gezielter auf die Bedürfnisse eines Patienten ausgerichtet werden, indem durch eine HIS-Bündel-Stimulation das bestehende Reizsystem des Herzens H zusätzlich für eine synchrone Anregung im Bereich des linken Ventrikels LV und des rechten Ventrikels RV ausgenutzt wird.

Bei dem in Fig. 3 dargestellten Ausführungsbeispiel ist der erste Steckverbinder 21 als IS4-Stecker mit vier elektrischen Kontaktelementen K1-K4 ausgebildet, die zur elektrischen Kontaktierung mit den Kontaktelementen A1-A4 des Steckverbinderanschlusses 103 des Anschlussblocks 101 des Pulsgenerators 10 dienen.

Die zweiten Steckverbinder 22, 23 sind demgegenüber jeweils als zweipolige IS-1-Anschlüsse mit zwei Kontaktelementen A1, A2 ausgebildet. An die zweiten Steckverbinder 22, 23 kann je eine Elektrode 12A, 12B mit einem als zweipoliger IS-1-Stecker ausgebildeten Gegensteckverbinder 122A, 122B angeschlossen werden. In verbundenem Zustand kontaktieren Kontaktelemente K1, K2 der Gegensteckverbinder 122A, 122B mit den Kontaktelementen A1, A2 des jeweils zugeordneten zweiten Steckverbinders 22, 23.

Die Adaptereinrichtung 2 dient dazu, die elektrische Verbindung eines einzelnen Steckverbinderanschlusses 103 auf Seiten des Anschlussblocks 101 elektrisch auf mehrere zweite Steckverbinder 22, 23 zu verteilen. Wie dies in Fig. 4 dargestellt ist, ist ein jedes Kontaktelement A1, A2 eines jeden zweiten Steckverbinders 22, 23 über eine in der Adaptereinrichtung 2 eingefasste elektrische Leitung L1-L4 mit einem zugeordneten der Kontaktelemente K1-K4 des ersten Steckverbinders 21 elektrisch verbunden.

Beispielsweise können die Kontaktelemente K1, K2 des ersten Steckverbinders 21 über Leitungen L1, L2 mit den Kontaktelementen A1, A2 des zweiten Steckverbinders 22 elektrisch verbunden sein. Die Kontaktelemente K3, K4 des ersten Steckverbinders 21 sind demgegenüber über Leitungen L3, L4 elektrisch mit den Kontaktelementen A1, A2 des zweiten Steckverbinders 23 verbunden.

Auch andere Ausgestaltungen sind denkbar und möglich. Beispielsweise können die Kontaktelemente K3, K4 des ersten Steckverbinders 21 mit den Kontaktelementen A1, A2 des zweiten Steckverbinders 22 verbunden sein, während die Kontaktelemente K1, K2 des ersten Steckverbinders 21 mit den Kontaktelementen A1, A2 des zweiten Steckverbinders 23 verbunden sind.

Bei dem in Fig. 3 und 4 dargestellten Ausführungsbeispiel sind die zweiten Steckverbinder 22, 23 jeweils als zweipolige Anschlüsse ausgestaltet. Dies ist jedoch nicht zwingend.

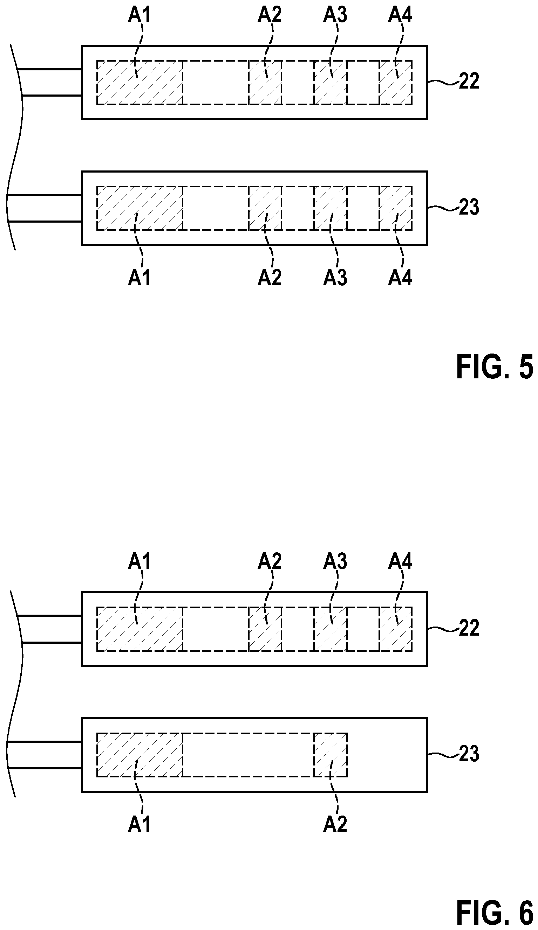

Beispielsweise können die Steckverbinder 22, 23 auch jeweils als vierpolige Anschlüsse in Form von IS4-Anschlüssen mit jeweils vier Kontaktelementen A1-A4 ausgebildet sein, wie dies in Fig. 5 dargestellt ist.

Alternativ kann ein Anschluss 22, 23 als vierpoliger IS4-Anschluss und der andere Anschluss 23, 22 als zweipoliger IS-1-Anschluss ausgebildet sein, wie dies schematisch in einem Beispiel in Fig. 6 dargestellt ist.

Bei den vierpoligen Steckverbindern 22, 23 des Beispiels gemäß Fig. 5 sind beispielsweise zwei der Kontaktelemente A1-A4 der Steckverbinder 22, 23 jeweils mit zwei zugeordneten Kontaktelementen K1-K4 des Steckverbinders 21 verbunden, während die anderen beiden Kontaktelemente A1-A4 des jeweiligen Steckverbinders 22, 23 elektrisch nicht angebunden sind. Alternativ können die Kontaktelemente A1-A4 der Steckverbinder 22, 23 jeweils paarweise mit einem zugeordneten Kontaktelement K1-K4 des Steckverbinders 21 verbunden sein, sodass jeweils zwei Kontaktelemente A1-A4 eines jeden Steckverbinders 22, 23 mit einem gemeinsamen Kontaktelement K1-K4 auf Seiten des Steckverbinders 21 elektrisch verbunden sind.

Entsprechend kann dies bei dem vierpoligen Steckverbinder 22 des Beispiels gemäß Fig. 6 ausgeführt sein.

Durch Verwendung einer Adaptereinrichtung 2 der beschriebenen Art werden neue Therapieformen möglich, im Rahmen derer bei einer kardialen Resynchronisationstherapie eine HIS-Bündel-Stimulation mit einbezogen werden kann. Eine Anpassung des Pulsgenerators 10 eines bestehenden CRT-Systems 1 ist hierbei nicht zwingend erforderlich. Durch Verwendung der Adaptereinrichtung 2 können bei einem bestehenden Pulsgenerator 10 Elektroden 12A, 12B flexibel über die Adaptereinrichtung 2 angeschlossen werden, um durch Verwendung dieser Elektroden 12A, 12B zum Beispiel sowohl eine linksventrikuläre Stimulation als auch eine HIS-Bündel-Stimulation zu ermöglichen, wie dies in Fig. 7 dargestellt ist.

Bei einem in Fig. 1 dargestellten, bestehenden Pulsgenerator 10 werden über die Steuereinrichtung 105 elektrische Stimulationspulse generiert und über die Steckverbinderanschlüsse 102, 103, 104 an die zugeordneten Elektroden 11, 12, 13 abgegeben. Bei einem vierpoligen Steckverbinderanschluss 103, 104 für eine linksventrikuläre Elektrode 12 oder eine rechtsventrikuläre Elektrode 13 können hierbei in mehrpoliger Weise unterschiedliche zeitversetzte Stimulationspulse generiert und abgegeben werden, wobei eine zeitliche Verzögerung zwischen zum Beispiel den linksventrikulären Stimulationspulsen einstellbar ist. Bei Verwendung der Adaptereinrichtung 2 kann über eine in einem bestehenden Pulsgenerator 10 bestehende Möglichkeit zur Programmierung einer solchen zeitlichen Verzögerung auch ein Zeitversatz zwischen einer HIS-Bündel-Stimulation über die Elektrode 12B und einer linksventrikulären Stimulation über die Elektrode 12A eingestellt werden, sodass der bestehende Pulsgenerator 10 ohne aufwendige Anpassung weiterhin verwendet werden kann.

Die Adaptereinrichtung 2 kann an ihren Steckverbindern 22, 23 zum Kennzeichnen der anzuschließenden Elektroden 12A, 12B markiert sein (beispielsweise mit "LV" und "HIS").

Eine durch Software verwirklichte Programmiergeräte-Oberfläche für den Pulsgenerator 10 kann angepasst werden, sodass in der Programmiergeräte-Oberfläche die entsprechend anzuschließenden Elektroden 12A, 12B ("LV", "HIS") gekennzeichnet sind.

Das CRT-System 1 kann als reines Schrittmachersystem ausgebildet sein (sogenanntes CRT-P-System). Alternativ kann das CRT-System auch eine Defibrillator-Funktion aufweisen (sogenanntes CRT-D-System).

Der der Erfindung zugrunde liegende Gedanke ist nicht auf die vorangehend beschriebenen Ausführungsbeispiele beschränkt, sondern lässt sich auch in anderer Weise verwirklichen. Die Anschlüsse des Pulsgenerators können auch anders als beschrieben ausgebildet sein. Beispielsweise kann der Pulsgenerator lediglich zwei oder auch mehr als drei Anschlüsse aufweisen.

Die Adaptereinrichtung kann mehr als zwei zweite Steckverbinder, beispielsweise drei oder vier zweite Steckverbinder aufweisen, sodass mehr als zwei Elektroden an die Adaptereinrichtung angeschlossen werden können.

### Liste der Bezugszeichen

- 1: CRT-System
- 10: Pulsgenerator
- 100: Gehäuse
- 101: Anschlussblock
- 102-104: Steckverbinderanschluss
- 105: Steuereinrichtung
- 106: Energieversorgungseinrichtung
- 11: Elektrode
- 110: Distales Ende
- 111: Proximales Ende
- 112: Gegensteckverbinder
- 12, 12A, 12B: Elektrode
- 120, 120A, 120B: Distales Ende
- 121, 121A, 121B: Proximales Ende
- 122, 122A, 122B: Gegensteckverbinder
- 13: Elektrode
- 130: Distales Ende
- 131: Proximales Ende
- 132: Gegensteckverbinder
- 2: Adaptereinrichtung
- 20: Verbindungsstück
- 21-23: Steckverbinder
- A1-A4: Kontaktelement
- AVN: AV-Knoten
- H: Herz
- HIS: HIS-Bündel
- K1-K4: Kontaktelement
- L1-L4: Elektrische Leitung
- LA: Linkes Atrium
- LBB: Linker Bündelzweig
- LV: Linkes Ventrikel
- M: Herzgewebe (Myokard)
- RA: Rechtes Atrium
- RBB: Rechter Bündelzweig
- RV: Rechtes Ventrikel
- SN: Sinusknoten
- V: Obere Hohlvene

## Patentansprüche

1. CRT-System (1) zur kardialen Resynchronisationstherapie, mit einem Pulsgenerator (10), der einen Anschlussblock (101) mit einer Mehrzahl von Steckverbinderanschlüssen (102-104) aufweist, und einer Mehrzahl von an die Steckverbinderanschlüsse (102-104) anschließbaren Elektroden (11, 12, 12A, 12B, 13), die jeweils einen Gegensteckverbinder (112, 122, 122A, 122B, 132) aufweisen, **gekennzeichnet durch** eine Adaptereinrichtung (2), die einen ersten Steckverbinder (21) und zumindest zwei zweite Steckverbinder (22, 23) aufweist, wobei der erste Steckverbinder (21) steckend mit einem der Steckverbinderanschlüsse (102-104) des Anschlussblocks (101) des Pulsgenerators (10) und die zweiten Steckverbinder (22, 23) jeweils steckend mit einem Gegensteckverbinder (112, 122, 122A, 122B, 132) einer der Mehrzahl von Elektroden (11, 12, 12A, 12B, 13) verbindbar sind.

2. CRT-System (1) nach Anspruch 1, **dadurch gekennzeichnet, dass** der erste Steckverbinder (21) erste elektrische Kontaktelemente (K1-K4) zum elektrischen Kontaktieren mit Kontaktelementen (A1-A4) des einen der Steckverbinderanschlüsse (102-104) des Anschlussblocks (101) des Pulsgenerators (10) aufweist.

3. CRT-System (1) nach Anspruch 2, **dadurch gekennzeichnet, dass** der erste Steckverbinder (21) vier elektrische Kontaktelemente (K1-K4) aufweist.

4. CRT-System (1) nach Anspruch 2 oder 3, **dadurch gekennzeichnet, dass** der erste Steckverbinder (21) ein IS4-Stecker ist.

5. CRT-System (1) nach einem der vorangehenden Ansprüche, **dadurch gekennzeichnet, dass** die zweiten Steckverbinder (22, 23) jeweils zweite elektrische Kontaktelemente (A1-A4) zum elektrischen Kontaktieren mit Kontaktelementen (K1-K4) des Gegensteckverbinders (112, 122, 122A, 122B, 132) der zugeordneten Elektrode aufweisen.

6. CRT-System (1) nach Anspruch 5, **dadurch gekennzeichnet, dass** zumindest einer der zweiten Steckverbinder (22, 23) zwei zweite elektrische Kontaktelemente (A1-A4) aufweist.

7. CRT-System (1) nach Anspruch 6, **dadurch gekennzeichnet, dass** der zumindest eine der zweiten Steckverbinder (22, 23) ein IS-1-Anschluss ist.

8. CRT-System (1) nach einem der Ansprüche 5 bis 7, **dadurch gekennzeichnet, dass** zumindest einer der zweiten Steckverbinder (22, 23) vier zweite elektrische Kontaktelemente (A1-A4) aufweist.

9. CRT-System (1) nach Anspruch 8, **dadurch gekennzeichnet, dass** der zumindest eine der zweiten Steckverbinder (22, 23) ein IS4-Anschluss ist.

10. CRT-System (1) nach einem der Ansprüche 5 bis 9, **dadurch gekennzeichnet, dass** zumindest einige der zweiten elektrischen Kontaktelemente (A1-A4) der zweiten Steckverbinder (22, 23) mit zugeordneten ersten elektrischen Kontaktelementen (K1-K4) des ersten Steckverbinders (21) elektrisch verbunden sind.

11. CRT-System (1) nach einem der vorangehenden Ansprüche, **dadurch gekennzeichnet, dass** an einen der zweiten Steckverbinder (22, 23) eine Elektrode zur HIS-Bündel-Stimulation und an einen anderen der zweiten Steckverbinder (22, 23) eine Elektrode zur linksventrikulären Stimulation anschließbar ist.

12. CRT-System (1) nach einem der vorangehenden Ansprüche, **dadurch gekennzeichnet, dass** die Adaptereinrichtung (2) gemeinsam mit dem Pulsgenerator (10) und den Elektroden (11, 12, 12A, 12B, 13) implantierbar ist.

13. Verfahren zur Durchführung einer kardialen Resynchronisationstherapie unter Verwendung eines CRT-Systems (1), aufweisend: Bereitstellen eines Pulsgenerators (10) des CRT-Systems (1), der einen Anschlussblock (101) mit einer Mehrzahl von Steckverbinderanschlüssen (102-104) aufweist; Bereitstellen einer Mehrzahl von an die Steckverbinderanschlüsse (102-104) anschließbaren Elektroden (11, 12, 12A, 12B, 13), die jeweils einen Gegensteckverbinder (112, 122, 122A, 122B, 132) aufweisen; Verbinden eines ersten Steckverbinders (21) einer Adaptereinrichtung (2) mit einem der Steckverbinderanschlüsse (102-104) des Anschlussblocks (101) des Pulsgenerators (10); und Verbinden der Gegensteckverbinder (112, 122, 122A, 122B, 132) von zumindest zwei der Mehrzahl von Elektroden (11, 12, 12A, 12B, 13) mit zweiten Steckverbindern (22, 23) der Adaptereinrichtung (2).

14. Verfahren nach Anspruch 13, **dadurch gekennzeichnet, dass** eine erste Elektrode (12B) der Mehrzahl von Elektroden (11, 12, 12A, 12B, 13) als HIS-Bündel-Elektrode mit einem distalen Ende (120B) im Bereich des HIS-Bündels (HIS) eines Patienten implantiert wird.

15. Verfahren nach Anspruch 14, **dadurch gekennzeichnet, dass** zur Durchführung der kardialen Resynchronisationstherapie eine zweite Elektrode (11) der Mehrzahl von Elektroden (11, 12, 12A, 12B, 13) mit ihrem distalen Ende (110) im rechten Atrium (RA), eine dritte Elektrode (12A) der Mehrzahl von Elektroden (11, 12, 12A, 12B, 13) mit ihrem distalen Ende (120A) im linken Ventrikel (LV) und/oder eine vierte Elektrode (13) der Mehrzahl von Elektroden (11, 12, 12A, 12B, 13) mit ihrem distalen Ende (130) im rechten Ventrikel (RV) implantiert werden.
